# EUROPEAN PATENT SPECIFICATION

(11) **EP 3 182 980 B1**
(45) Date of publication and mention of the grant of the patent: **14.10.2020**
(21) Application number: 15833822.8
(22) Date of filing: 19.08.2015
(51) Int. Cl.: A61K 31/7072, A61K 31/7004, A61K 31/7008, A61K 31/7012, A61P 43/00, A61P 21/00, A61K 45/06

(54) **TREATMENT OF GLYCOSYLATION DEFICIENCY DISEASES**
BEHANDLUNG VON GLYCOSYLIERUNGSMANGELKRANKHEITEN
TRAITEMENT DE MALADIES LIÉES À UNE DÉFICIENCE DE LA GLYCOSYLATION

(30) Priority: 19.08.2014 US 201462039052 P
(43) Date of publication of application: 28.06.2017
(73) Proprietor: Wellstat Therapeutics Corporation, Rockville, MD 20850 (US)
(72) Inventor: VON BORSTEL, Reid W., Potomac, MD 20854 (US)
(74) Representative: Titmus, Craig Edward
(86) International application number: PCT/US2015/045896
(87) International publication number: WO 2016/028894

(56) References cited:
- WO-A1-2013/102904
- US-A1- 2011 257 233
- US-A1- 2011 257 233
- US-A1- 2012 078 529
- US-A1- 2012 078 529
- T Marquardt ET AL: "Congenital disorders of glycosylation: review of their molecular bases, clinical presentations and specific therapies", European Journal of Pediatrics, 1 June 2003 (2003-06-01), pages 359-379, XP055470970, Berlin/Heidelberg DOI: 10.1007/s00431-002-1136-0 Retrieved from the Internet: URL:https://link.springer.com/content/pdf/ 10.1007/s00431-002-1136-0.pdf

## Description

### BACKGROUND OF THE INVENTION

Congenital disorders of glycosylation (CDG) encompass a group of genetic diseases caused by defects in enzymes of glycosylation pathways mediating synthesis of oligosaccharides on glycoproteins or glycolipids. Many glycosylation reactions use uridine diphosphate (UDP) as a sugar transfer molecule, with intermediates such as UDP-glucose, UDP-glucosamine, UDP-galactose, UDP galactosamine, UDP-mannose, UDP-mannosamine and other nucleotide sugars. Such glycosylation disorders include but are not limited to those reviewed in Freeze H (2013) JBC 288(210):6936-6945. Additional specific glycosylation disorders are identified frequently, as analytical and diagnostic techniques improve. Glycosylation defects addressable by the methods and compositions of this disclosure can comprise deficits in synthesis of specific sugars or sugar nucleotides, or in deficits in enzymes catalyzing glycosylation reactions. Glycosylation defects can be due to activity-reducing mutations affecting enzyme structure and function or to reduced expression of active enzymes.

### SUMMARY OF THE INVENTION

The invention is defined according to the claims. Thus, in one embodiment, there is provided a uridine prodrug for use in treating a genetic glycosylation disorder in a subject, wherein said treating comprises administration of the uridine prodrug to the subject in an amount sufficient to raise plasma uridine in the subject to a level greater than 30 micromolar; wherein the uridine prodrug is uridine triacetate; and wherein: (a) said treating comprises administration of the uridine prodrug in one or more doses from 0.5 to 5 grams of the uridine prodrug per square meter of body surface area; optionally wherein the uridine prodrug dose is from 1.5 to 3 grams per square meter of body surface area; (b) the uridine prodrug is formulated for administration in one or more doses from 0.5 to 5 grams of the uridine prodrug per square meter of body surface area; optionally wherein the uridine prodrug dose is from 1.5 to 3 grams per square meter of body surface area; or (c) said treating comprises administration of the uridine prodrug in one or more doses from 2 to 10 grams.

In one embodiment, the invention provides a pharmaceutical composition for use in treating a genetic glycosylation disorder in a subject, the composition comprising a uridine prodrug in an amount sufficient to raise plasma uridine in the subject to a level greater than 30 micromolar; wherein the uridine prodrug is uridine triacetate; and wherein: (a) said treating comprises administration of the pharmaceutical composition in one or more doses from 0.5 to 5 grams of the uridine prodrug per square meter of body surface area; optionally wherein the uridine prodrug dose is from 1.5 to 3 grams per square meter of body surface area; (b)the pharmaceutical composition is formulated for administration in one or more doses from 0.5 to 5 grams of the uridine prodrug per square meter of body surface area; optionally wherein the uridine prodrug dose is from 1.5 to 3 grams per square meter of body surface area; or (c) said treating comprises administration of the uridine prodrug in one or more doses from 2 to 10 grams.

In one embodiment, the uridine prodrug or the pharmaceutical composition according to the invention is for use in treating a Type II congenital disorder of glycosylation.

In one embodiment, the uridine prodrug or the pharmaceutical composition according to the invention is for use in treating a Type I congenital disorder of glycosylation or a Type III congenital disorder of glycosylation.

In one embodiment, treatment of genetic glycosylation disorder according to the invention comprises administration of the uridine triacetate in one, two, three, or four daily doses.

In one embodiment, the level of plasma uridine in the subject is from 45 micromolar to 55 micromolar.

In one embodiment, the subject is a human subject.

In one embodiment, treatment of genetic glycosylation disorder according to the invention further comprises administration of an effective amount of a specific sugar, transfer of which is defective in the glycosylation disorder.

In one embodiment, the glycosylation disorder is a GNE myopathy and the specific sugar is sialic acid, mannosamine, or N-acetylmannosamine; or the glycosylation disorder is a PGM-1 deficiency and the specific sugar is d-galactose; or the glycosylation disorder is GPT deficiency and the specific sugar is N-acetylglucosamine.

Described herein is a method of treating a genetic glycosylation disorder in a subject, comprising administering to the subject a uridine prodrug in an amount sufficient to raise plasma uridine in the subject to a level greater than 30 micromolar. Also described herein is a uridine prodrug for use in treating a genetic glycosylation disorder in a subject, wherein the uridine prodrug is administered to the subject in an amount sufficient to raise plasma uridine in the subject to a level greater than 30 micromolar. Also described herein is the use of a uridine prodrug in the manufacture of a medicament for treating a genetic glycosylation disorder in a subject, wherein the medicament is formulated for administration in an amount sufficient to raise plasma uridine in the subject to a level greater than 30 micromolar. Also described herein is a pharmaceutical composition for use in treating a genetic glycosylation disorder in a subject, the composition comprising a uridine prodrug in an amount sufficient to raise plasma uridine in the subject to a level greater than 30 micromolar.

### DETAILED DESCRIPTION OF THE INVENTION

In diseases with a defect in a pathway in which a glycosylation reaction involving a UDP-sugar (or in the case of sialic acid, a cytidine monophosphate (CMP)-sugar) are at fault, production of the deficient nucleotide sugar (pyrimidine-phospho-sugar) via residual, but inadequate, enzyme activity is augmented by precursor loading, i.e. increasing intracellular uridine triphosphate (UTP) and the specific sugar or its precursor, to form increased intracellular concentrations of the UDP-sugar.

Extracellular uridine concentrations sufficient to increase intracellular UTP and UDP sugars are required, and are in the range of >30 micromolar steady-state concentration, advantageously >50 micromolar while at the same time increasing availability of the specific sugar. Sugar availability may be increased by either administration of the specific sugar or a precursor that is efficiently converted to it, or by inhibiting pathways involved in sugar degradation, or both administering a sugar or precursor and blocking degradation pathways.

Uridine is advantageously administered to patients in the form of an orally bioavailable prodrug which, according to the present invention, is uridine triacetate (2',3',5'-tri-O-acetyluridine). An appropriate single dose is in the range of 2 to 10 grams, administered once to four times daily depending on need and response.

The specific sugars, examples of which are glucosamine, N-acetylglucosamine, galactose, N-acetylgalactose, mannose, or N-acetylgalactose, precursors of these or other relevant sugars, or inhibitors of their catabolism, are administered in doses sufficient to raise their intracellular concentrations in tissues affected by the specific glycosylation disorder.

CDG often affect the development and function of the brain, but also frequently affect peripheral organs as well. A wide variety of symptoms can be caused by glycosylation disorders, including developmental delays (both cognitive and physical), seizures, ataxia, hypotonia, seizures, retinal problems, liver abnormalities including fibrosis, impaired hematopoiesis, and structural malformations. Definitive diagnosis generally requires identification of a pathogenic mutation in a glycosylation pathway, which provides the requisite information for determining whether uridine loading is likely to be beneficial.

CDG have classically been divided into two main groups based on diagnosis using transferrin glycosylation patterns. Type I CDG affects the early steps in oligosaccharide synthesis, production of lipid-linked oligosaccharides in the endoplasmic reticulum. Type II glycosylation disorders comprise defects in glycosylation that occur after glycans are added to proteins, and affect subsequent elongation, trimming and processing of the attached glycans. More recently, CDG that do not fit into either of these categories have been identified, affecting non-glycan glycosylation of proteins. See Freeze H (2013) JBC 288(210):6936-6945, and the references cited therein. Marquardt T et al. (European Journal of Pediatrics (2003), 162:359-379) disclose the therapeutic options available for the treatment of CDGs.

Pyrimidine-phospho-sugar precursors are involved in subsets of both Type I and Type II CDG, and in glycosylation disorders not falling into either of these categories which will be referred to herein as Type III glycosylation disorders. ("Type I" CDG and "Type II" CDG are art-recognized names for these categories of CDG, whereas the name "Type III" CDG is used in this document for convenience.) PGM deficiency (see below and example 3) and GPT deficiency (see example 4) are examples of Type II CDG. GNE myopathy (see below and example 2) is primarily a Type II CDG but also extends to Type III CDG.

The typical dose for uridine prodrugs in general and uridine triacetate in particular is an amount sufficient to achieve steady state average plasma concentration >30 micromolar, but levels as high as about 50 micromolar (e.g., from 45 to 55 micromolar) may be necessary for some difficult patients. Appropriate doses of uridine triacetate to achieve steady state plasma uridine >30 micromolar are 0.5 to 5 grams per square meter of body surface area (BSA), advantageously 1.5 to 3 grams per square meter of BSA. BSA is determined using standard drug dosing tables or formulas, using body weight, height, sex and age as input variables. Doses are administered orally one to four times per day, approximately evenly spaced throughout the 24 hour day.

The distinguishing characteristic of CDG that are beneficially treated with a uridine precursor and sugar or sugar precursor are those in which the biochemical lesion comprises a deficiency in production or availability (including transport deficits reducing pyrimidine nucleotide sugar delivery into the endoplasmic reticulum) of a pyrimidine nucleotide sugar, or those in which enzyme activity using a pyrimidine nucleotide sugar as a substrate are low, and thereby contributing to disease pathogenesis.

Glucosamine (UDP-N-acetyl)-2-epimerase/N-acetylmannosamine kinase (GNE) is an enzyme involved in synthesis of sialic acid, a sugar found in many glycoproteins. GNE myopathy, or hereditary inclusion body myopathy is caused by a defect in production of sialic acid, a precursor for synthesis of sialoglycoconjugates. Exogenous sialic acid (or sialic acid prodrugs or precursors such as mannosamine or N-acetylmannosamine) is being tested as a treatment, bypassing the enzymatic defect. The agents and methods of this disclosure augments the efficacy of exogenous sialic acid by increasing intracellular pyrimidine nucleotides, including cytidine triphosphate (CTP), which is derived from uridine triphosphate (UTP). Increased intracellular CTP improves the efficiency of conversion of exogenous sialic acid to CMP-sialic acid, the nucleotide sugar used for transfer of sialic acid onto growing oligosaccharides. Appropriate doses of sialic acid or N-acetylmannosamine for treatment of GNE myopathy are 3 to 10 grams per day, depending on the severity of the molecular lesion and the size of the patient.

Phosphoglucomutase-1 (PGM-1) deficiency is a CDG involving insufficient production of galactose, with multisystem symptoms that may include growth delay, malformations like cleft palate, hypoglycemia, and liver and heart dysfunction. Supplementary galactose at doses of 0.5 to 1 g/kg per day is used for treatment, with partial resolution of some symptoms. Uridine triacetate administered concurrently with galactose augments the therapeutic efficacy of galactose in this disease.

Several known CDG feature defects in N-glycan synthesis and processing. Type II CDG are often associated with developmental delays, hypotonia, seizures, and organ dysfunction. For these Type II CDG including CPT deficiency, N-acetylglucosamine is a potential treatment, given in doses up to 200 mg/kg/day. In these patients, uridine triacetate is co-administered with N-acetylglucosamine to enhance its efficacy in restoring N-glycan synthesis and processing.

In other glycosylation disorders in which the production, utilization, transport or availability of a pyrimidine nucleotide sugar contributes to disease pathogenesis, uridine triacetate is administered to enhance the efficacy of the particular monosaccharide deemed appropriate for that specific CDG, and the monosaccharide is administered in daily doses that are considered appropriate for it as monotherapy.

The subject that can be treated in accordance with this invention is any animal, whether vertebrate or invertebrate, but is preferably a mammalian subject including a human subject.

The invention will be better understood by reference to the following examples, which illustrate but do not limit the invention described herein.

### EXAMPLES

### EXAMPLE 1: Treatment of a CDG with Uridine triacetate

A patient displaying developmental delays is diagnosed with a congenital disorder of glycosylation by detection of a mutation affecting glycosylation by reducing availability of a uridine-diphospho sugar, corroborated by biochemical measurements in cells from the patient. The patient is treated with oral uridine triacetate at a dose of 2 grams per square meter of body surface area, administered three times per day. The patient responds to treatment, displaying biochemical measures of improved protein glycosylation and concurrent improvements in clinical condition.

### EXAMPLE 2: GNE Myopathy

A patient displaying progressive distal muscle weakness, with vacuoles and filamentous inclusions in a muscle biopsy, is diagnosed with GNE myopathy by testing for mutations in GNE, which result in impaired synthesis of endogenous sialic acid. The patient is treated with oral N-acetylmannosamine at a dose of 3 to 10 grams per day. The patient is also treated with uridine triacetate at a dosage of 2 grams per square meter of body surface area administered twice per day. The uridine triacetate improves the clinical response beyond that achieved with N-acetylmannosamine alone.

### EXAMPLE 3: Phosphoglucomutase-1 Deficiency (Type II CDG)

A patient with multisystem disease consistent with phosphoglucomutase deficiency receives a definitive diagnosis via detection of impaired transferring glycosylation and a mutation in PGM-1. The patient is treated with 1 gram/kg of d-galactose per day, and with uridine triacetate at a dosage of 2 grams per square meter of body surface area administered twice per day The uridine triacetate improves the clinical response beyond that achieved with d-galactose alone.

### EXAMPLE 4: UDP-GlcNAc: dolichol phosphate N-acetyl-glucosamine-1 phosphate transferase (GPT) deficiency (Type II CDG)

A patient displaying severe hypotonia, medically intractable seizures, developmental delay and microcephaly is diagnosed with a deficiency of activity of the enzyme GPT, a Type II CDG, by detection of a mutation in its encoding gene DPAGT-1. ("GPT" is the enzyme dolichol phosphate N-acetyl-glucosamine-1 phosphate transferase; "DPAGT-1" is the gene that encodes GPT.) The patient is treated with N-acetylglucosamine at a dose of 200 mg/kg per day. The patient is also treated with uridine triacetate at a dosage of 2 grams per square meter of body surface area administered twice per day. The uridine triacetate improves the clinical response beyond that achieved with N-acetylglucosamine alone.

## Claims

1. A uridine prodrug for use in treating a genetic glycosylation disorder in a subject, wherein said treating comprises administration of the uridine prodrug to the subject in an amount sufficient to raise plasma uridine in the subject to a level greater than 30 micromolar; wherein the uridine prodrug is uridine triacetate; and wherein:
(a) said treating comprises administration of the uridine prodrug in one or more doses from 0.5 to 5 grams of the uridine prodrug per square meter of body surface area; optionally wherein the uridine prodrug dose is from 1.5 to 3 grams per square meter of body surface area;
(b) the uridine prodrug is formulated for administration in one or more doses from 0.5 to 5 grams of the uridine prodrug per square meter of body surface area; optionally wherein the uridine prodrug dose is from 1.5 to 3 grams per square meter of body surface area; or
(c) said treating comprises administration of the uridine prodrug in one or more doses from 2 to 10 grams.

2. A pharmaceutical composition for use in treating a genetic glycosylation disorder in a subject, the composition comprising a uridine prodrug in an amount sufficient to raise plasma uridine in the subject to a level greater than 30 micromolar; wherein the uridine prodrug is uridine triacetate; and wherein:
(a) said treating comprises administration of the pharmaceutical composition in one or more doses from 0.5 to 5 grams of the uridine prodrug per square meter of body surface area; optionally wherein the uridine prodrug dose is from 1.5 to 3 grams per square meter of body surface area;
(b) the pharmaceutical composition is formulated for administration in one or more doses from 0.5 to 5 grams of the uridine prodrug per square meter of body surface area; optionally wherein the uridine prodrug dose is from 1.5 to 3 grams per square meter of body surface area; or
(c) said treating comprises administration of the uridine prodrug in one or more doses from 2 to 10 grams.

3. The uridine prodrug for use according to claim 1, or the pharmaceutical composition for use according to claim 2, wherein the genetic glycosylation disorder is a Type II congenital disorder of glycosylation.

4. The uridine prodrug for use according to claim 1, or the pharmaceutical composition for use according to claim 2, wherein the genetic glycosylation disorder is a Type I congenital disorder of glycosylation or a Type III congenital disorder of glycosylation.

5. The uridine prodrug for use according to claim 1 or the pharmaceutical composition for use according to claim 2, wherein said treating comprises administration of the uridine triacetate in one, two, three, or four daily doses.

6. The uridine prodrug for use according to claim 1, or the pharmaceutical composition for use according to claim 2, wherein the level of plasma uridine in the subject is from 45 micromolar to 55 micromolar.

7. The uridine prodrug for use according to claim 1, or the pharmaceutical composition for use according to claim 2, wherein the subject is a human subject.

8. The uridine prodrug for use according to claim 1, or the pharmaceutical composition for use according to claim 2, wherein said treatment further comprises administration of an effective amount of a specific sugar, transfer of which is defective in the glycosylation disorder.

9. The uridine prodrug for use or the pharmaceutical composition for use according to claim 8, wherein the glycosylation disorder is a GNE myopathy and the specific sugar is sialic acid, mannosamine, or N-acetylmannosamine; or the glycosylation disorder is a PGM-1 deficiency and the specific sugar is d-galactose; or the glycosylation disorder is GPT deficiency and the specific sugar is N-acetylglucosamine.

## Patentansprüche

1. Uridin-Prodrug zur Verwendung beim Behandeln einer genetischen Glykosylierungsstörung bei einem Probanden, wobei das Behandeln eine Verabreichung des Uridin-Prodrugs an den Probanden in einer Menge umfasst, die ausreicht, um das Plasma-Uridin in dem Probanden auf einen Spiegel von mehr als 30 Mikromol zu erhöhen; wobei das Uridin-Prodrug Uridintriacetat ist; und wobei:
(a) das Behandeln eine Verabreichung des Uridin-Prodrugs in einer oder mehreren Dosen von 0,5 bis 5 Gramm des Uridin-Prodrugs pro Quadratmeter Körperoberfläche umfasst; optional wobei die Uridin-Prodrug-Dosis von 1,5 bis 3 Gramm pro Quadratmeter Körperoberfläche beträgt;
(b) das Uridin-Prodrug zur Verabreichung in einer oder mehreren Dosen von 0,5 bis 5 Gramm des Uridin-Prodrugs pro Quadratmeter Körperoberfläche formuliert wird; optional wobei die Uridin-Prodrug-Dosis von 1,5 bis 3 Gramm pro Quadratmeter Körperoberfläche beträgt; oder
(c) das Behandeln eine Verabreichung des Uridin-Prodrugs in einer oder mehreren Dosen von 2 bis 10 Gramm umfasst.

2. Pharmazeutische Zusammensetzung zur Verwendung beim Behandeln einer genetischen Glykosylierungsstörung bei einem Probanden, wobei die Zusammensetzung ein Uridin-Prodrug in einer Menge umfasst, die ausreicht, um das Plasma-Uridin in dem Probanden auf einen Spiegel von mehr als 30 Mikromol zu erhöhen; wobei das Uridin-Prodrug Uridintriacetat ist; und wobei:
(a) das Behandeln eine Verabreichung der pharmazeutischen Zusammensetzung in einer oder mehreren Dosen von 0,5 bis 5 Gramm des Uridin-Prodrugs pro Quadratmeter Körperoberfläche umfasst; optional wobei die Uridin-Prodrug-Dosis von 1,5 bis 3 Gramm pro Quadratmeter Körperoberfläche beträgt;
(b) die pharmazeutische Zusammensetzung zur Verabreichung in einer oder mehreren Dosen von 0,5 bis 5 Gramm des Uridin-Prodrugs pro Quadratmeter Körperoberfläche formuliert wird; optional wobei die Uridin-Prodrug-Dosis von 1,5 bis 3 Gramm pro Quadratmeter Körperoberfläche beträgt; oder
(c) das Behandeln eine Verabreichung des Uridin-Prodrugs in einer oder mehreren Dosen von 2 bis 10 Gramm umfasst.

3. Uridin-Prodrug zur Verwendung nach Anspruch 1 oder pharmazeutische Zusammensetzung zur Verwendung nach Anspruch 2, wobei die genetische Glykosylierungsstörung eine angeborene Glykosylierungsstörung vom Typ II ist.

4. Uridin-Prodrug zur Verwendung nach Anspruch 1 oder pharmazeutische Zusammensetzung zur Verwendung nach Anspruch 2, wobei die genetische Glykosylierungsstörung eine angeborene Glykosylierungsstörung vom Typ I oder eine angeborene Glykosylierungsstörung vom Typ III ist.

5. Uridin-Prodrug zur Verwendung nach Anspruch 1 oder pharmazeutische Zusammensetzung zur Verwendung nach Anspruch 2, wobei das Behandeln eine Verabreichung des Uridintriacetats in einer, zwei, drei oder vier Tagesdosen umfasst.

6. Uridin-Prodrug zur Verwendung nach Anspruch 1 oder pharmazeutische Zusammensetzung zur Verwendung nach Anspruch 2, wobei der Plasma-Uridin-Spiegel in dem Probanden von 45 Mikromol bis 55 Mikromol beträgt.

7. Uridin-Prodrug zur Verwendung nach Anspruch 1 oder pharmazeutische Zusammensetzung zur Verwendung nach Anspruch 2, wobei der Proband ein menschlicher Proband ist.

8. Uridin-Prodrug zur Verwendung nach Anspruch 1 oder pharmazeutische Zusammensetzung zur Verwendung nach Anspruch 2, wobei die Behandlung weiterhin eine Verabreichung einer wirksamen Menge eines spezifischen Zuckers, dessen Transfer in der Glykosylierungsstörung mangelhaft ist, umfasst.

9. Uridin-Prodrug zur Verwendung oder pharmazeutische Zusammensetzung zur Verwendung nach Anspruch 8, wobei die Glykosylierungsstörung eine GNE-Myopathie ist und der spezifische Zucker Sialinsäure, Mannosamin oder N-Acetylmannosamin ist; oder die Glykosylierungsstörung ein PGM-1-Mangel ist und der spezifische Zucker D-Galaktose ist; oder die Glykosylierungsstörung GPT-Mangel ist und der spezifische Zucker N-Acetylglukosamin ist.

## Revendications

1. Promédicament d'uridine destiné à être utilisé dans le traitement d'un trouble génétique de la glycosylation chez un sujet, dans lequel ledit traitement comprend l'administration du promédicament d'uridine au sujet dans une quantité suffisante pour augmenter l'uridine plasmatique chez le sujet jusqu'à un niveau supérieur à 30 micromolaires ; dans lequel le promédicament d'uridine est le triacétate d'uridine ; et dans lequel :
(a) ledit traitement comprend l'administration du promédicament d'uridine en une ou plusieurs doses de 0,5 à 5 grammes du promédicament d'uridine par mètre carré de surface corporelle ; facultativement dans lequel la dose de promédicament d'uridine est de 1,5 à 3 grammes par mètre carré de superficie corporelle ;
(b) le promédicament d'uridine est formulé pour une administration en une ou plusieurs doses de 0,5 à 5 grammes du promédicament d'uridine par mètre carré de superficie corporelle ; facultativement dans lequel la dose de promédicament d'uridine est de 1,5 à 3 grammes par mètre carré de superficie corporelle ; ou
(c) ledit traitement comprend l'administration du promédicament d'uridine en une ou plusieurs doses de 2 à 10 grammes.

2. Composition pharmaceutique destinée à être utilisée dans le traitement d'un trouble génétique de la glycosylation chez un sujet, la composition comprenant un promédicament d'uridine dans une quantité suffisante pour augmenter l'uridine plasmatique chez le sujet jusqu'à un niveau supérieur à 30 micromolaires ; dans laquelle le promédicament d'uridine est le triacétate d'uridine ; et dans laquelle :
(a) ledit traitement comprend l'administration de la composition pharmaceutique en une ou plusieurs doses de 0,5 à 5 grammes du promédicament d'uridine par mètre carré de surface corporelle ; facultativement dans laquelle la dose de promédicament d'uridine est de 1,5 à 3 grammes par mètre carré de superficie corporelle ;
(b) la composition pharmaceutique est formulée pour une administration en une ou plusieurs doses de 0,5 à 5 grammes du promédicament d'uridine par mètre carré de superficie corporelle ; facultativement dans laquelle la dose de promédicament d'uridine est de 1,5 à 3 grammes par mètre carré de superficie corporelle ; ou
(c) ledit traitement comprend l'administration du promédicament d'uridine en une ou plusieurs doses de 2 à 10 grammes.

3. Promédicament d'uridine destiné à être utilisé selon la revendication 1, ou composition pharmaceutique destinée à être utilisée selon la revendication 2, dans lequel le trouble génétique de la glycosylation est un trouble congénital de la glycosylation de Type II.

4. Promédicament d'uridine destiné à être utilisé selon la revendication 1, ou composition pharmaceutique destinée à être utilisée selon la revendication 2, dans lequel le trouble génétique de la glycosylation est un trouble congénital de la glycosylation de Type I ou un trouble congénital de la glycosylation de Type III.

5. Promédicament d'uridine destiné à être utilisé selon la revendication 1 ou composition pharmaceutique destinée à être utilisée selon la revendication 2, dans lequel ledit traitement comprend l'administration du triacétate d'uridine en une, deux, trois, ou quatre doses quotidiennes.

6. Promédicament d'uridine destiné à être utilisé selon la revendication 1, ou composition pharmaceutique destinée à être utilisée selon la revendication 2, dans lequel le niveau d'uridine plasmatique chez le sujet est de 45 micromolaires à 55 micromolaires.

7. Promédicament d'uridine destiné à être utilisé selon la revendication 1, ou composition pharmaceutique destinée à être utilisée selon la revendication 2, dans lequel le sujet est un sujet humain.

8. Promédicament d'uridine destiné à être utilisé selon la revendication 1, ou composition pharmaceutique destinée à être utilisée selon la revendication 2, dans lequel ledit traitement comprend en outre l'administration d'une quantité efficace d'un sucre spécifique, dont le transfert est déficient dans le trouble de la glycosylation.

9. Promédicament d'uridine destiné à être utilisé ou composition pharmaceutique destinée à être utilisée selon la revendication 8, dans lequel le trouble de la glycosylation est une myopathie liée à GNE et le sucre spécifique est l'acide sialique, la mannosamine, ou la N-acétylmannosamine ; ou le trouble de la glycosylation est une déficience en PGM-1 et le sucre spécifique est le d-galactose ; ou le trouble de la glycosylation est la déficience en GPT et le sucre spécifique est la N-acétylglucosamine.
